# EUROPEAN PATENT SPECIFICATION

(11) **EP 1 608 346 B1**
(45) Date of publication and mention of the grant of the patent: **12.11.2008**
(21) Application number: 04759681.2
(22) Date of filing: 19.03.2004
(51) Int. Cl.: A61K 9/48

(54) **ALPHA-HYDROXY ACID ESTER DRUG DELIVERY COMPOSITIONS AND METHODS OF USE**
ZUSAMMENSETZUNGEN MIT ALPHAHYDROXY-SÄUREESTER ZUR WIRKSTOFFFREISETZUNG UND ANWENDUNGSVERFAHREN DAFÜR
COMPOSITIONS POUR L'ADMINISTRATION DE MEDICAMENTS CONTENANT UN ESTER D'ALPHA-HYDROXY-ACIDE ET METHODES D'UTILISATION DE CES COMPOSITIONS

(30) Priority: 31.03.2003 US 403071
(43) Date of publication of application: 28.12.2005
(73) Proprietor: ABBOTT LABORATORIES, Abbott Park IL 60064-3500 (US)
(72) Inventor: LIPARI, John, M., Racine, WI 53406 (US); LONG, Michelle, A., Libertyville, IL 60048 (US)
(74) Representative: Modiano, Micaela Nadia
(86) International application number: PCT/US2004/008417
(87) International publication number: WO 2004/093851

(56) References cited:
- WO-A-00/67728
- WO-A-01/82903
- WO-A-98/17288
- WO-A-99/01120
- WO-A-99/29316
- WO-A-03/011258
- GESTMANN A ET AL: "TESTING A NEW PLATOFRM FOR SKIN CARE EMULSIONS" COSMETICS & TOILETRIES, WHEATON, IL, US, vol. 117, no. 9, September 2002 (2002-09), pages 73-74,76,78, XP008025627 ISSN: 0361-4387

## Description

### Field of the Invention

The invention generally relates to carrier compositions, and methods of using such, useful for the delivery of a pharmaceutical drug active or prodrug to a subject. More specifically, the carrier composition includes an alpha-hydroxy acid ester and acylglycerols which provide excellent drug solubility properties and a pH sensitivity for the improved delivery of the pharmaceutical drug active to the subject.

### Background

Various factors influence the bioavailability of drugs following oral administration. These include drug solubility, stability of the drug following administration, and the absorption of the drug from the gastrointestinal (GI) tract, among other factors. The drug delivery system (i.e., the delivery vehicle or composition coadministered with the drug) can affect these factors, and in particular, the absorption of the drug from the GI tract. In particular, formulating drug delivery systems for hydrophobic drugs presents a greater challenge due to their poor solubility and bioavailability, in general. Delivery systems have been developed that contain surfactant and/or lipid-based excipients to increase the solubility or improve the dissolution rate of drugs, and thus improve their absorption from the gastrointestinal tract. However, many of these delivery systems are not completely effective in delivering a desired dose to the subject or result in unwanted side effects upon ingestion (e.g., the delivery system is not well-tolerated in the GI tract). Absorption of the drug active or prodrug also may be variable from dose to dose, resulting in an unpredictable bioavailability. In addition to these factors, the synthesis of pharmaceutical drug actives or prodrugs can be expensive and therefore maximizing the bioavailability of a drug through the use of a novel delivery system is desirable. Techniques and compositions are sought in order to overcome these problems associated with the oral delivery of drugs and to increase the bioavailability of the administered compound and reduce the patient-to-patient blood level variability.

International Patent Application WO 99/01120 A discloses the use of IMWITOR 375 as a solubilizer for sertraline in an amount of about 1 % by weight of the test solution.

There is a need for drug delivery systems that increase the absorption and thus bioavailability of pharmaceutical drug actives or prodrugs, in particular hydrophobic pharmaceutical drug actives or prodrugs.

### Summary

The invention provides pharmaceutical compositions comprising a carrier composition and a pharmaceutical drug active or prodrug useful for delivery of the drug to a subject. The carrier composition includes a delivery composition that has an alpha-hydroxy acid ester and an acylglycerol. The alpha-hydroxy acid ester is composed basically of an alpha-hydroxy acid portion, a glycerol portion, and a fatty acid portion. The alpha-hydroxy acid ester also is found at more than 36%, as measured by percentage weight/weight (w/w), in the carrier composition.

In some embodiments the alpha-hydroxy acid ester is derived from a monoacylglycerol and the acylglycerol component is a monoacylglycerol. In some embodiments the carrier composition includes a cosolvent. The cosolvent can either be lipophilic, hydrophilic, or a mixture thereof.

In another embodiment the carrier composition consists essentially of the delivery composition and the cosolvent.

The invention also provides for pharmaceutical capsules having a capsule shell, a fill composition, and a pharmaceutical drug active or prodrug. The fill composition comprises a delivery composition that has an alpha-hydroxy acid ester and an acylglycerol. The alpha-hydroxy acid ester comprises an alpha-hydroxy acid portion, a glycerol portion, and a fatty acid portion. The alpha-hydroxy acid ester is also found at more than 36% w/w in the carrier composition.

In one embodiment the alpha-hydroxy acid ester is unneutralized.

The invention also provides for methods of delivering a pharmaceutical drug active or prodrug to a subject using the delivery composition. In one embodiment the pharmaceutical drug active or prodrug is delivered orally to the subject in a carrier composition which includes the delivery composition. The unique pH sensitivity of the alpha-hydroxy acid ester allows the pharmaceutical composition to demonstrate lipophilic properties in a low pH environment, such as in the stomach or other medium with a pH of less than pH 4, and surface active properties in a higher pH environment, such as the intestinal tract or other medium with a pH of pH 4 or greater.

### Brief Description of the Figures

Figure 1 represents the equilibrium phase behavior for an example delivery composition (Imwitor^{™} 380, unneutralized) at different concentrations in 0.1 *N* HCl.
Figure 2 represents the equilibrium phase behavior for an example delivery composition (Imwitor^{™} 380, unneutralized) at different concentrations in pH 7.4 phosphate buffer.

### Detailed Description

### Overview

The invention provides compositions and methods for the delivery of a pharmaceutical drug active or a prodrug to a subject. Generally, the invention provides a pharmaceutical composition that includes a carrier composition for pharmaceutical drug actives or prodrugs. The carrier composition incorporates, at least in part, a delivery composition. The delivery composition primarily comprises an alpha-hydroxy acid ester and an acylglycerol. According to the invention, the alpha-hydroxy acid ester content in the carrier composition is more than 36% as measured by percentage weight/weight relative to the weight of the carrier composition. A pharmaceutical drug active or prodrug is dissolved or suspended in the carrier composition to provide a useful pharmaceutical composition, which can be administered to a subject, typically by oral administration. The pharmaceutical composition includes a pharmaceutical drug active, or prodrug, typically dissolved or suspended in the carrier composition and encapsulated in a soft or hard capsule shell.

The carrier composition of the invention provides an advantageous delivery system for the administration of a pharmaceutical drug active or prodrug. The carrier composition is suitable for suspending or solubilizing a plurality of pharmaceutical drug actives or prodrugs. In particular, the carrier composition provides an excellent delivery system for hydrophobic drugs.

The delivery composition, which includes the alpha-hydroxy acid ester and the acylglycerol, demonstrates properties that have been discovered to promote the absorption of the pharmaceutical drug active or prodrug from the gastrointestinal tract. The unique properties of the delivery composition are particularly useful for delivery of a hydrophobic drug to a subject. Therefore, the properties of the delivery composition allow for the preparation of solutions or suspensions of a pharmaceutical drug active or prodrug, and delivery of the drug and carrier composition to a subject, typically by oral administration.

Although not intending to be limited by theory, it is believed that the delivery composition demonstrates a pH sensitivity that results in variable phase behavior. The variable phase behavior can be promoted by changes in environmental conditions *in vivo* (i.e., varying pH conditions throughout the gastrointestinal tract). In low pH environments (viz., in the stomach), the delivery composition, which includes the alpha-hydroxy acid ester and the acylglycerol, is essentially lipophilic and displays the properties of a neutral oil. Upon an increase in pH (viz., in the intestinal tract), the delivery composition becomes surface active and spontaneously disperses, forming, for example, small spherical structures. Accordingly, the delivery composition provides a method for the enhanced absorption of a pharmaceutical drug active or prodrug in a subject.

As used herein, "pharmaceutical drug active", "drug active", or "drug" refers to one or more, or a combination of different compounds beneficial for the treatment or prevention of a disease or physiological condition. The compound or compounds can be administered to an organism and can modify or alter one or more aspects of the organism's physiology. The term "prodrug" as used herein refers to a compound, or a metabolite thereof, useful for the treatment or prevention of disease or a physiological condition. In one embodiment of the invention the pharmaceutical drug active or prodrug is an orally-delivered compound that becomes systemic via the gastrointestinal tract. In other embodiments the pharmaceutical drug active or prodrug is administered topically or parenterally administered. More specifically, it refers to any compound intended for use in the treatment or prevention of disease. According to the invention, the pharmaceutical drug active or prodrug is solubilized or suspended in at least the delivery composition, which includes at least the alpha-hydroxy acid ester.

As used herein, "pharmaceutical composition" refers to a composition of matter containing at least one pharmaceutical drug active or prodrug that has a pharmacological activity and a carrier composition for the drug, the delivery composition portion of the carrier composition facilitating drug delivery to a subject via administration, including oral administration.

The terms "monoacylglycerols", "diacylglycerols", and "triacylglycerols" refer to mono-, di-, and triesters, respectively, of glycerol with fatty acids, compounds which can be found in, or derived from, various edible fats and oils. As used herein, "acylglycerols" collectively refers to monoacylglycerols, diacylglycerols, and triacylglycerols.

### Delivery and Carrier Compositions

As used herein, "delivery composition" refers to a composition that includes an alpha-hydroxy acid ester component and an acylglycerol component and that is suitable for solubilizing or suspending a pharmaceutical drug active or prodrug for delivery to a subject. In some embodiments the delivery composition is at least a portion of a carrier composition. In these embodiments the carrier composition can include other components, such as, for example, cosolvents. In another embodiment the carrier composition consists entirely of the components of the delivery composition.

The delivery composition portion of the carrier composition includes an alpha-hydroxy acid ester and an acylglycerol. In one embodiment, the alpha-hydroxy acid ester is primarily formed from monoacylglycerols and the acylglycerol component of the delivery composition is primarily monoacylglycerols. In other embodiments the alpha-hydroxy acid ester can be formed from other acylglycerols, for example, diacylglycerols. Typically, the delivery composition also includes glycerol which can be a byproduct in the starting materials used in the reaction between alpha-hydroxy acids with acylglycerols. Therefore, the delivery composition consists essentially of an alpha-hydroxy acid ester and an acylglycerol and, in some cases, an amount of glycerol or other reaction products associated with the synthesis of the alpha-hydroxy acid ester.

In one embodiment, the delivery composition portion of the carrier composition includes an alpha-hydroxy acid ester, and the alpha-hydroxy acid ester is more than 36% w/w of the carrier composition. In some embodiments, the alpha-hydroxy acid ester content is in the range of 36-60% w/w in the carrier composition. In other embodiments the alpha-hydroxy acid ester content is greater than 60% w/w in the carrier composition. It is within the skill of one in the formulation arts to adjust the alpha-hydroxy acid ester content within these ranges to provide an appropriate pharmaceutical composition depending on various factors, for example, the amount and type of pharmaceutical drug active or prodrug used in the composition. Addition of other components to the carrier composition, for example, cosolvents also can affect the concentration of the alpha-hydroxy acid ester in the carrier composition.

In some embodiments the carrier composition consists entirely of the delivery composition. In these embodiments, and using commercial preparations of alpha-hydroxy acid esters, for example, citric or lactic acid esters, the amount of alpha-hydroxy acid ester in the carrier composition can be at least 60% w/w.

In some embodiments commercial preparations of alpha-hydroxy acid ester can be used and the alpha-hydroxy acid ester content in the delivery composition is typically at least 60% w/w. In these preparations an acylglycerol also is a component of the delivery composition. Typically, the acylglycerol content in the delivery composition is not greater than 35% w/w. The acyl glycerol typically includes mono- and diacylglycerols. More typically, the acyl glycerol is primarily monoacylglycerols. In some embodiments, the delivery composition includes reacted and unreacted products of the esterification of mono- ° and diacylglycerols of saturated and unsaturated edible fatty acids with an alpha-hydroxy acid. In addition, unreacted glycerol can be present in the delivery composition. Glycerol is typically found at 5% w/w or less in the delivery composition.

In some embodiments, cosolvents are present in the carrier composition. The addition of cosolvents to the carrier composition can enhance the properties of the pharmaceutical composition, for example, by improving pharmaceutical drug active or prodrug solubility. The cosolvent can include hydrophilic cosolvents, lipophilic cosolvents, and mixtures thereof. Examples of suitable hydrophilic cosolvents include ethanol, benzyl alcohol, propylene glycol, polyethylene glycols, glycerin, water, and N-methyl-2-pyrrolidone, or a mixture thereof. Examples of suitable lipophilic cosolvents include triacetin, ethyl oleate, long chain alcohols, for example C₁₂-C₂₄ alcohols, and isopropyl myristate, or a mixture thereof. Preferably a cosolvent is used that is miscible with the delivery composition.

In some embodiments, the carrier composition includes a delivery composition, a cosolvent, and other components. In these embodiments, the cosolvent can be at any concentration provided that the alpha-hydroxy acid ester content in the carrier composition is more than 36% w/w. A useful range for the cosolvent is between 10-40% w/w in the carrier composition or from 5-20% w/w of the total pharmaceutical composition.

The pharmaceutical composition also optionally can include other adjuvants, for example, opacifiers, dyes or excipients, for example, flavors, sweeteners, thickeners, and preservatives, such as antioxidants. Such adjuvants can be suspended or solubilized in the carrier composition or the delivery composition.

### Alpha-Hydroxy Acid Ester

One component of the delivery composition of the carrier composition is an alpha-hydroxy acid ester. Alpha-hydroxy acid esters typically include, but are not limited to, alpha-hydroxy acid esters of monoacylglycerols as represented by Formulas I-III (alpha-hydroxy acid esters having the fatty acid portion at the G₁ position are shown), as shown below, wherein "X" represents a fatty acid portion of the alpha-hydroxy acid ester, "Y" represents the alpha-hydroxy acid portion of the alpha-hydroxy acid ester, and wherein both the fatty acid portion "X" and the alpha-hydroxy acid portion "Y" are covalently attached to the glycerol portion by an ester bond. "OH" or "HO" represents the unreacted hydroxyl group of the glycerol portion. G₁, G₂, and G₃ represent carbon positions on the glycerol portion of the alpha-hydroxy acid ester. The term "glycerol portion" refers to the part of the alpha-hydroxy acid ester that serves as the backbone for attachment of one or more "fatty acid portions" or the "alpha-hydroxy acid portion"; the glycerol portion is separated from the fatty acid portions and alpha-hydroxy acid portion by an ester bond. The fatty acid portion "X", the alpha-hydroxy acid portion "Y", and the hydroxyl group "OH" can be pendant from any carbon position G₁, G₂, or G₃ on the glycerol portion of the alpha-hydroxy acid ester. Compounds of these formulas can be prepared, for example, by the reaction of monoacylglycerols, or mixtures of monoacylglycerols, with one or more alpha-hydroxy acids.

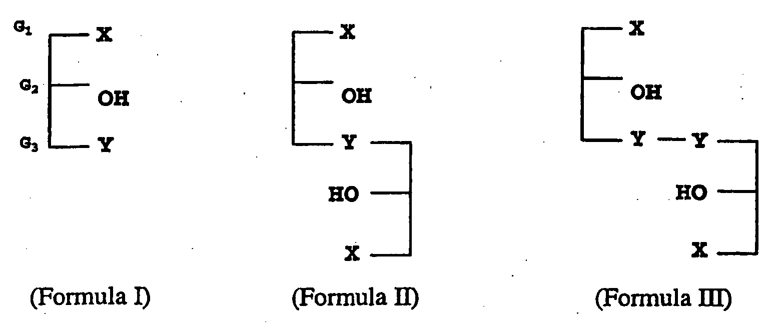

Alpha-hydroxy acid esters can also typically include compounds as defined by Formula II wherein the alpha-hydroxy acid portion "Y" is able to form ester bonds with two glycerol portions having ester-bonded fatty acid portions. For compounds of Formula II, the alpha-hydroxy acid ester typically has two or more groups capable of reacting with the hydroxyl groups of two or more monoacylglycerols. In compounds of Formula II, the fatty acid portions "X" can be the same or different, as the alpha-hydroxy acid ester can be formed from mixtures of different monoacylglycerols. Alpha-hydroxy acid esters also can typically include compounds defined by Formula III. These compounds can be formed by the dimerization of two alpha-hydroxy acid esters, for example, by the formation of an ester bond between a hydroxyl group of an alpha-hydroxy acid portion of the molecule and the carboxylic acid group of another alpha-hydroxy acid portion of the molecule. The delivery composition can include any compound as defined by Formulas I-III or mixtures thereof. One with skill in the formulation arts also would understand that by using oils or fats as a source of different monoacylglycerols, a plurality of alpha-hydroxy acid esters can be obtained having any one of the Formulas I-III, or combinations thereof.

In addition, monoacylglycerols can exist as 2-monoacylglycerols, as defined by the fatty acid portions being pendant from the G₂ carbon positions on the glycerol portion of the molecule. It is understood that the presence of additional species of alpha-hydroxy acid esters can exist based on the positioning of the fatty acid portion in 2-monoacylglycerols.

An example of an alpha-hydroxy acid ester according to the invention, 3-carboxy-3-hydroxy-pentanedioic acid mono-(2-hydroxy-3-octadecanoyloxy-propyl) ester, which, in one synthetic method, can be formed by the esterification of citric acid with monostearoylglycerol, is shown as Compound I.

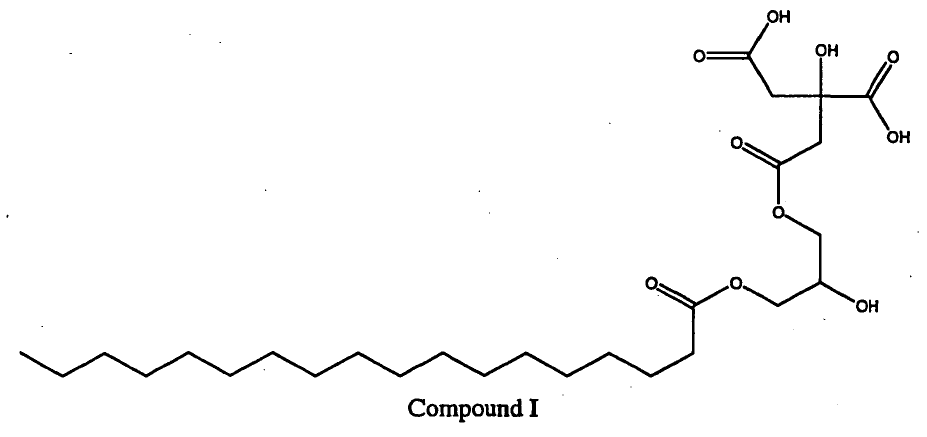

In addition, monoacylglycerols can exist as 1-monoacylglycerols or 2-monoacylglycerols, as defined by the fatty acid portion being pendant from either the G₁/G₃ or G₂ carbon on the glycerol portion of the molecule. It is understood that the presence of mixtures of 1- and 2-monoacylglycerols, for example, those obtained from oils or fats for the preparation of the alpha-hydroxy acid ester, further increases the plurality of different alpha-hydroxy acid esters in the delivery composition.

The delivery composition also can include alpha-hydroxy acid esters formed by the reaction of diacylglycerols, or mixtures of diacylglycerols, with one or more alpha-hydroxy acids. Alpha-hydroxy acid esters also typically can include, but are not limited to, the compounds represented by Formulas IV-VI (alpha-hydroxy acid esters having the fatty acid portions in the G₁ and G₂ positions are shown).

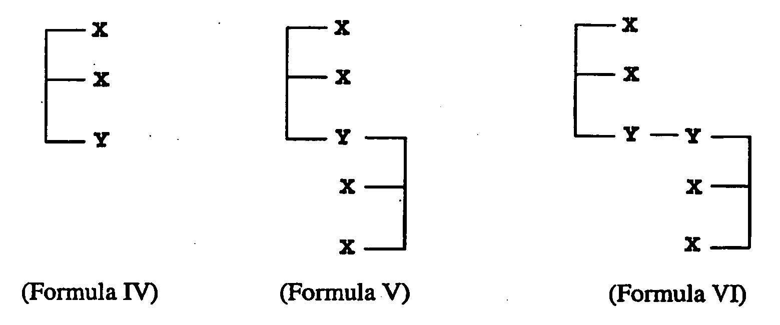

The fatty acid portions "X" can be the same or different, as the alpha-hydroxy acid ester can be formed from diacylglycerols having the same or different fatty acid portions. The delivery composition can include compounds having any one of the Formulas IV-VL It is understood that, by using oils or fats as a source of different diacylglycerols, a plurality of alpha-hydroxy acid esters can be obtained having any one of the Formulas IV-VI, or combinations thereof.

In addition, diacylglycerols can exist as 1,3-diacylglycerols, as defined by the fatty acid portions being pendant from the G₁ and G₃ carbon positions on the glycerol portion of the molecule. One with skill in the formulation arts would understand that the presence of additional species of alpha-hydroxy acid esters can exist based on the positioning of the fatty acid portions in 1,3-diacylglycerols. The presence of mixtures of different diacylglycerols, for example, present as starting materials in, or derived from, oils or fats for the preparation of the alpha-hydroxy acid ester, further increases the plurality of different alpha-hydroxy acid esters in the delivery composition portion of the carrier composition.

The invention also contemplates delivery compositions comprising mixtures of alpha-hydroxy acid esters formed from the esterification of monoacylglycerols and diacylglycerols with alpha-hydroxy acids. The delivery composition can include alpha-hydroxy acid esters formed from the esterification of an alpha-hydroxy acid with any combination of 1-monoacylglycerols, 2-monoacylglycerols, 1,2-diacylglycerols and 1,3-diacylglycerols.

At the "X" position of the alpha-hydroxy acid esters, the fatty acid portion can include saturated and mono- and polyunsaturated fatty acids. In one embodiment of the invention, the fatty acids are of medium chain and long chain length, for example, fatty acids chains having a length in the range of C₆ to C₂₄. In a preferred embodiment, the fatty acid chain lengths of the alpha-hydroxy acid ester are in the range of C₈ to C₁₈.

Suitable fatty acid portions of the alpha-hydroxy acid ester include straight or branched chain, saturated or unsaturated fatty acids such as, for example, butyric acid, caproic acid, caprylic acid, capric acid, lauric acid, myristic acid, palmitic acid, palmitoleic acid, stearic acid, isostearic acid, arachidonic acid, cetic acid, linoleic acid, eicosanoic acid, oleic acid, and linolenic acid.

Suitable fatty acids for the preparation of the alpha-hydroxy acid esters include fatty acids commonly obtained from natural plant and animal oils, for example, peanut oil, rapeseed oil, soybean oil, palm oil, cottonseed oil, olive oil, sunflower seed oil, corn/maize oil, palm kernel oil, coconut oil, marine oils, such as fish oil, animal fats, specialty oils, and the like. Preferably, the alpha-hydroxy acid esters are formed from mono- and diacylglycerols derived from coconut oil or soybean oil. The use of fatty acids from sources other than natural plant and animal oils, such as synthetic fatty acids, are also contemplated.

Preferred fatty acids, or mixtures of fatty acids, include those high in lauric acid (C₁₂) content, which are typically derived from coconut oil. A high percentage of triacylglycerols in coconut oil have C₁₂ fatty acid chains in all three positions on the glycerol backbone. (It is known that various oils contain mixtures of mono-, di-, and triacylglycerols, such information can be found in, for example, Fennema, O.R., Food Chemistry, Third Edition, p. 238, 1996, ed.: Marcel Dekker, Inc., New York.) Moderate amounts of shorter chain fatty acids, for example, C₆, C₈, and C₁₀ fatty acids, are also derived from coconut oils, and can optionally be fractionated therefrom. Other preferred fatty acids are derived from cocoa butter, which has a high percentage of C₁₆ and C₁₈ saturated fatty acid chains at the 1 and 3 carbon positions on the glycerol portion and a high percentage of C₁₈ unsaturated fatty acid chains at the 2 carbon position on the glycerol portion. Other preferred oil sources for the preparation of alpha-hydroxy acid esters include acylglycerols from palm kernel and soybean oil. These, or other oils or fats, can be used as starting materials for reaction with one or more alpha-hydroxy acids to achieve the desired alpha-hydroxy acid ester or mixture of alpha-hydroxy acid esters for the delivery composition.

The delivery composition can be formulated and prepared to have certain physical properties, for example, to exist in a liquid or solid state at a desired condition. Therefore, a suitable oil, oil mixture, fat, or fat mixture, can be utilized for preparation of the delivery composition. Selection of oils or fats for the preparation of the alpha-hydroxy acid esters can be based on the melting temperature of the oils or fats. For example, it may be desirable to utilize an alpha-hydroxy acid ester prepared from fats and oils having a low melting temperature if a temperature-sensitive pharmaceutical drug active or prodrug is to be solubilized or suspended.

According to the invention, the alpha-hydroxy acid ester can be formed from the esterification of monoacylglycerols or diacylglycerols, or mixtures thereof, with one or more alpha-hydroxy acids. Typically, the alpha-hydroxy acid ester is formed from primarily monoacylglycerols, which can be accomplished by a variety of methods, for example, by the reaction of oil or fat preparations, or mixtures thereof, with one or more alpha-hydroxy acid. In one embodiment, the alpha-hydroxy acid ester is formed from monoacylglycerols from coconut, palm kernel, or soybean oil. In a particularly useful embodiment, the alpha-hydroxy acid ester is a citric or lactic acid ester of coconut, palm kernel, or soybean oil. Optionally, the alpha-hydroxy acid ester can be formed by the synthesis of monoacylglycerols, and optionally diacylglycerols, from glycerol and one or more fatty acids and the subsequent reaction of the monoacylglycerol (or diacylglycerol) with one or more alpha-hydroxy acids.

Preparation of the starting materials for the synthesis of the alpha-hydroxy acid ester can be performed in several ways, for example, by chemical deacylation (e.g., with a Grignard reagent, e.g., ethyl magnesium bromide) which can produce monoacylglycerols and diacylglycerols from triacylglycerols. In another method, enzymatic deacylation of the primary ester bonds of triacylglycerols by use of pancreatic lipase can be used to form monoacylglycerols and diacylglycerols. Another method for the preparation of acylglycerols is the reaction of fatty acids, for example, obtained from a suitable oil source or purchased commercially (e.g., from Sigma, St. Louis, MO), with glycerol. Optionally, particular acylglycerols can be fractionated or purified from an oil or fat source. Synthesis of acylglycerols from glycerol and purified or commercially purchased fatty acids can allow the user to control with precision the fatty acid content and identity of the alpha-hydroxy acid ester.

As used herein, the terms "free glycerol", and "free nionoacylglycerols and diacylglycerols", or "free acylglycerols", refers to components in the delivery composition that have not undergone further chemical reactions. These free components can be unreacted products in the formation of the alpha-hydroxy acid ester. In particular, the unreacted "free" acylglycerols can be, and are typically, the acylglycerol component in the delivery composition.

As used herein, "alpha-hydroxy acids" generally refer to organic acids wherein at least one hydroxyl group is present on the carbon adjacent to an acid functional group (i.e., carboxylic acid group). According to the invention, alpha-hydroxy acids (i.e., the alpha-hydroxy acid portion of the alpha-hydroxy acid ester) are exemplified by the following formula: (Rₐ)(R_{b})C(OH)COOH, wherein Rₐ and R_{b} are H, F, Cl, Br, alkyl, aralkyl, or aryl group of saturated, unsaturated, straight or branched chain or cyclic form having 1 to 10 carbon atoms, and in addition Rₐ or R_{b} may carry one or more OH, CHO, COOH or alkoxy groups having 1 to 9 carbon atoms as well as mixtures of two or more different types of such group.

Examples of alpha-hydroxy acids include lactic acid, glycolic acid, malic acid, tartronic acid, tartaric acid, glucuronic acid, 2-hydroxyisobutyric acid, 3-hydroxybutyric acid, citric acid, galacturonic acid, mandelic acid, alpha-phenylpyruvic acid, saccharic acid, alpha-hydroxyisocaproic acid, alpha-hydroxy-isovaleric acid, atrolactic acid, glyceric acid, isocitric acid, dihydroxymaleic acid, dihydroxytartaric acid, and dihydroxy-fumaric acid. Citric acid and lactic acid are the preferred alpha-hydroxy acids for the alpha-hydroxy acid portion of the alpha-hydroxy acid ester.

Alpha-hydroxy acid esters can also be prepared in partially neutralized or unneutralized states. As used herein, "partially neutralized" refers to alpha-hydroxy acid esters that have been partially neutralized by the addition of a basic compound, for example, a basic salt, such as to the alpha-hydroxy acid ester reaction products, whereas "unneutralized" alpha-hydroxy acid esters do not have the addition of a basic compound. In some embodiments of the invention, partially neutralized alpha-hydroxy acid esters can be used to increase the hydrophilic properties of the delivery composition.

In some embodiments, the alpha-hydroxy acid portion of the alpha-hydroxy acid ester is citric acid or lactic acid, the fatty acid portion of the alpha-hydroxy acid ester contains fatty acids found in coconut, palm kernel, or soybean oil, or fatty acids having C₆ to C₂₄ chains, more preferably C₈ to C₁₈ chains, and the acylglycerol component of the delivery composition is primarily monoacylglycerols from coconut, palm kernel, or soybean oil, or acylglycerols having C₆ to C₂₄ chains, more preferably C₈ to C₁₈ chains.

Examples of materials suitable for the delivery composition can include commercially available products such as Imwitor^{™} 380 and 377, which are partially neutralized citric acid/lactic acid esters of partial glycerides derived from coconut oil; Imwitor^{™} 375, which is a partially neutralized citric acid/lactic acid ester of linoleate and oleate-containing mono- and diglycerides; and Imwitor^{™} 370, which is a partially neutralized citric acid ester of stearate-containing mono- and diglycerides. Imwitor^{™} 380, 377, 375, and 370 are commercially available from Sasol (Witten, Germany). Imwitor^{™} 380, 377, 375, and 370 are also available as unneutralized versions (e.g., Imwitor^{™} 361, an unneutralized version of Imwitor^{™} 370).

According to the invention, it has been discovered that the alpha-hydroxy acid esters demonstrate a unique pH sensitivity that differentiates them among other compounds, such as surfactants that lack an alpha-hydroxy acid portion and an acylglycerol portion. In one aspect, the alpha-hydroxy acid ester provides unique properties that allow for the spontaneous dispersion and wetting of instant pharmaceutical compositions when delivered to the higher pH environment of the intestinal tract. In another aspect, the alpha-hydroxy acid esters behave as a neutral oil when in an unneutralized state. Yet in another aspect, carrier compositions including an alpha-hydroxy acid ester are visually turbid when dispersed in an aqueous diluent.

Therefore, in one embodiment, it is preferable to use an alpha-hydroxy acid ester as the primary delivery component in the carrier composition to obtain the beneficial properties of the delivery composition as previously described. In a particularly preferred embodiment, lactic or citric acid esters of acylglycerols are the primary delivery component of the carrier composition.

In addition, the presence of additional components in other embodiments in combination with the delivery composition, such as solvents and cosolvents, can be beneficial for providing a suitable carrier composition as well.

### Drug Solubility

A variety of pharmaceutical drug actives or prodrugs demonstrate sufficient solubility in the delivery composition, which includes the alpha-hydroxy acid ester and the acylglycerol. In particular, the carrier composition of the invention is able to solubilize of number of hydrophobic drugs at relatively high concentrations.

In one embodiment, the invention provides a pharmaceutical composition, which includes an orally-administered pharmaceutical drug active or prodrug, and a delivery composition portion of a carrier composition for the pharmaceutical drug active or prodrug, which includes an alpha-hydroxy acid ester and an acylglycerol. The pharmaceutical composition can be in a solid or liquid state and these physical properties are typically dictated by the choice of materials (i.e., fatty acid component of the alpha-hydroxy acid ester and the acylglycerol) used to prepare the delivery composition. In some embodiments, the pharmaceutical drug active or prodrug is a hydrophobic drug.

### Pharmaceutical Capsules

The invention also provides pharmaceutical capsules which contain a fill composition. As used herein, "fill composition" refers to the contents of a pharmaceutical capsule surrounded by the capsule shell, which includes a carrier composition and the pharmaceutical drug active or prodrug. The fill composition includes a pharmaceutical drug active or prodrug and a delivery composition, as described herein. In some embodiments, the fill composition can include components in addition to the delivery composition, for example, cosolvents. In other embodiments, the fill composition is composed entirely of the delivery composition.

As used herein, "pharmaceutical capsule" refers to a conventional capsule for oral administration typically having a capsule shell containing the fill composition, wherein the capsule is capable of dissolving or disintegrating in the gastrointestinal tract to release the fill composition.

In one embodiment, the capsule shell is a soft elastic or hard capsule shell which can be prepared from, for example, gelatin, starch, or hydroxypropyl methylcellulose. Capsule shells can contain varying amounts of these materials and, optionally, other adjuvants, for example, plasticizers, opacifiers, dyes or excipients, for example, flavors, sweeteners, thickeners, and preservatives, such as antioxidants.

Various manufacturing processes that are well-known in the art can be used to form soft elastic or hard capsules from the fill and shell compositions described herein. Descriptions of these processes can be found in various references, for example, Principles and Practices of Pharmaceutics, The Pharmaceutical Codex, Twelfth Edition; Ed.: Walter Lund, The Pharmaceutical Press, London, 1994, p. 23-24 or Oral Solid Dosage Forms, Remington's Pharmaceutical Sciences, 17th Edition; Ed.: Gennaro, A.R., Mack Publishing Co., Easton, Pennsylvania, 1985, p. 1629-1631.

In one preferred embodiment of the invention, the pharmaceutical capsule includes a fill composition having a carrier composition including an unneutralized alpha-hydroxy acid ester.

### Pharmaceutical Drug Actives or Prodrugs

In some embodiments, and according to the invention, pharmaceutical drug actives or prodrugs can be orally-delivered and become available in the subject via the gastrointestinal tract. Pharmaceutical drug actives or prodrugs can modify or alter one or more aspects of the subject's physiology, and are generally intended for the treatment or prevention of disease. The amount of pharmaceutical drug active or prodrug present in the pharmaceutical composition or pharmaceutical capsule can be determined by a number of factors, including, for example, the solubility or suspendability of the drug in the carrier composition or the delivery composition, the activity of the drug following administration, and the absorption of the drug following administration.

The pharmaceutical drug actives or prodrugs can be solubilized or suspended in the carrier composition or delivery composition at a desired concentration. "Drug loading" refers to the amount of drug suspended or solubilized in the carrier composition.

In one embodiment of the invention, the pharmaceutical drug active or prodrug, for example, is a hydrophobic drug. As used herein, "hydrophobic drug" or "lipophilic drug" refer to one or more drugs, or combination of drugs, that display low solubility in a hydrophilic medium, and a higher solubility in hydrophobic mediums, for example organic solvents and the like. According to the invention, hydrophobic drugs are solubilized or dispersed by the carrier composition, which can include in whole or in part the delivery composition, and orally administered to a subject.

Hydrophobic drugs can include, but are not limited to, for example, the following classes of drugs: acetanilides, anilides, aminoquinolines, benzhydryl compounds, benzodiazepines, benzofurans, cannabinoids, cyclic peptides, dibenzazepines, digitalis glycosides, ergot alkaloids, flavonoids, imidazoles, quinolines, macrolides, naphthalenes, opiates (or morphinans), oxazines, oxazoles, phenylalkylamines, piperidines, polycyclic aromatic hydrocarbons, pyrrolidines, pyrrolidinones, stilbenes, sulfonylureas, sulfones, triazoles, tropanes, vinca alkaloids, and the like.

Specific examples of hydrophobic pharmaceutical drug actives or prodrugs include, but are not limited to the following compounds: acebutolol, acetazolamide, acetohexamide, acrivastine, albendazole, allopurinol, aloxiprin, alprazolam, alprenolol, amiloride, aminoglutethimide, amiodarone HCl, amlodipine, amodiaquine, amoxapine, amphetamine, amphotericin, amrinone, amsacrine, amyl nitrate, amylobarbitone, astemizole, atenolol, atropine, auranofin, azapropazone, azathioprine, barbitone, beclamide, beclomethasone, bendrofluazide, benethamine penicillin, benidipine, benorylate, bentazepam, benzhexol HCl, benznidazole, bephenium hydroxynaphthoate, betacarotene, betamethasone, bezafibrate, biperiden, bisacodyl, bromazepam, bromocriptine mesylate, bromperidol, brotizolam, budesonide, bumetanide, busulphan, butobarbitone, butoconazole nitrate, cambendazole, carbamazepine, carbimazole, carbromal, chlorambucil, chlordiazepoxide, chlormethiazole, chloroquine, chlorothiazide, chlorproguanil HCl, chlorpromazine, chlorpropamide, chlorthalidone, cimetidine, cinnarizine, cinoxacin, ciprofloxacin HCl, cisapride, clarithromycin, clioquinol, clobazam, clofazimine, clofibrate, clomiphene citrate, clonazepam, clotiazepam, clotrimazole, cloxacillin, clozapine, codeine, conjugated estrogens, cortisone acetate, cyclizine, cyclosporin, cyproheptadine HCl, dacarbazine, danazol, darodipine, decoquinate, demeclocycline, desoxymethasone, dexamethasone, dexamphetamine, dexfenfluramine, dextropropyoxyphene, diamorphine, diazepam, diazoxide, dichlorophen, dicoumarol, diflunisal, digitoxin, digoxin, dihydrocodeine, dihydroergotamine mesylate, diiodohydroxyquinoline, dilitazem HCl, diloxanide furoate, dimenhydrinate, dinitolmide, diphenoxylate HCl, dipyridamole, disopyramide, domperidone, doxycycline, droperidol, econazole nitrate, enoximone, ergotamine tartrate, erythromycin, estradiol, estramustine, ethacrynic acid, ethinamate, ethinyloestradiol, ethionamide, ethopropazine HCl, ethotoin, etodolac, etoposide, famotidine, felodipine, fenbufen, fenfluramine, fenofibrate, fenoprofen calcim, flecainide acetate, fluconazole, flucortolone, flucytosine, fludrocortisone acetate, flunanisone, flunarizine HCl, flunisolide, flunitrazepam, fluopromazine, flupenthixol decanoate, fluphenazine decanoate, flurazepam, flurbiprofen, fluticasone propionate, furosemide, furzolidone, gemfibrozil, glibenclamide, gliclazide, glipizide, glyceryl trinitrate, griseofulvin, guanabenz acetate, halofantrine HCl, haloperidol, hydrocortisone, hyoscyamine, ibuprofen, imipenem, indomethacin, isosorbide dinitrate, isosorbide mononitrate, isotretinoin, isradipine, itraconazole, ivermectin, ketoconazole, ketoprofen, labetalol, lanatoside C, lomustine, loperamide, loratadine, lorazepam, lormetazepam, lysuride maleate, maprotiline HCl, mazindol, mebendazole, meclofenamic acid, meclozine HCl, medazepam, medigoxin, medroxyprogesterone acetate, mefenamic acid, mefloquine HCl, melphalan, mepenzolate bromide, meprobamate, meptazinol, mercaptopurine, mesalazine, mestranol, methadone, methaqualone, methoin, methotrexate, methsuximide, methylphenobarbitone, methylprednisolone, methyltestosterone, methysergide maleate, metolazone, metoprolol, metronidazole, mianserin HCl, miconazole, midazolam, minoxidil, mitomycin, mitotane, mitozantrone, morphine, nabumetone, nadolol, nalbuphine, nalidixic acid, naproxen, natamycin, nicardipine HCl, nicoumalone, nifedipine, nimodipine, nimorazole, nitrazepam, nitrofurantoin, nitrofurazone, nizatidine, norethisterone, norgestrel, nortriptyline HCl, nystatin, omeprazole, ondansetron HCl, ornidazole, oxamniquine, oxantel embonate, oxatomide, oxazepam, oxcarbazepine, oxfendazole, oxprenolol, oxyphenbutazone, oxyphencylcimine HCl, paramethadione, pentaerythritol tetranitrate, pentazocine, pentobarbitone, perphenazine pimozide, phenacemide, phenindione, phenobarbitone, phenoxybenzamine HCl, phensuximide, phenylbutazone, phenytoin, pindolol, piroxicam, pizotifen maleate, praziquantel, prazosin HCl, prednisolone, prednisone, primidone, probenecid, probucol, procarbazine HCl, prochlorperazine, progesterone, proguanil HCl, propranolol, propylthiouracil, pyrantel embonate, pyrimethamine, quinidine sulphate, quinine sulphate, ranitidine HCl, reserpine, rifampicin, ritonavir, spiramycin, spironolactone, stanozolol, stiboestrol, sulconazole nitrate, sulindac, sulphabenzamide, sulphacetamide, sulphadiazine, sulphadoxine, sulphafurazole, sulphamerazine, sulphamethoxazole, sulphapyridine, sulphasalazine, sulphin-pyrazone, sulpiride, sulthiame, sumatriptan succinate, tamoxifen citrate, temazepam, terazosin HCl, terbinafine HCl, terconazole, terrenadine, testolactone, testosterone, tetracycline, thiabendazole, thioridazine, tibolone, tinidazole, tioconazole, tolazamide, tolbutamide, trazodone HCl, triamcinolone, triamterene, triazolam, trimethoprim, trimipramine maleate, tropicamide, undecenoic acid, valproic acid, vitamin A, vitamin B₂, vitamin D, vitamin E, vitamin K, and zopiclone.

### Treatment

In one aspect, the delivery composition portion of the carrier composition provides a particularly effective system for administration of hydrophobic or lipophilic pharmaceutical drug actives or prodrugs. Typically, the carrier composition having the pharmaceutical drug active or prodrug is delivered to a subject via oral administration.
According to the invention, it has been discovered that the delivery composition portion of the carrier composition, as described herein, displays properties that indicate the facilitation of delivery of a pharmaceutical drug active or prodrug to a subject. In one embodiment, the invention provides a method for the oral delivery of a pharmaceutical drug active or prodrug using a delivery composition having an alpha-hydroxy acid ester and an acylglycerol. In one aspect, the invention provides the discovery that the delivery composition has particular properties, for example, a useful pH sensitivity that in low pH environments, for example less than pH 4, which includes the stomach, the delivery composition displays properties of a neutral oil and does not readily disperse. Upon an increase in pH, for example, pH conditions found in the intestinal tract, the delivery composition becomes surface active and disperses. Higher pH conditions, for example pH 4 or greater, promote wetting and the formation of, for example, dispersed microspherical structures that promote the absorption of the pharmaceutical drug active or prodrug from the gastrointestinal tract.

The term "oral administration" refers to the process of taking pharmaceutical compositions or pharmaceutical capsules, as described herein, by mouth by a subject and also includes administrations by any type of gastrointestinal intubation including nasogastric or intestinal intubation. Oral administration can also include dilution of the pharmaceutical composition with other orally ingested materials, such as beverages, and oral consumption of the diluted pharmaceutical composition.

In one embodiment, the invention provides methods for the delivery of a pharmaceutical drug active or prodrug to a subject by the oral administration of the drug active or prodrug which has been suspended or solubilized in a carrier composition which incudes at least an alpha-hydroxy acid ester and an acylglycerol. In another embodiment, the invention provides methods for the delivery of the pharmaceutical drug active or prodrug by oral administration of a pharmaceutical capsule having a fill composition which includes the carrier composition and the drug active or prodrug. In other embodiments, the pharmaceutical composition may be delivered to the subject by other routes, for example, parenteral or topical administration.

The following examples are provided in reference to the invention as described.

### Example 1

### Solubility Screening

Various pharmaceutical drug actives and prodrugs were screened to test for solubility in the compositions including alpha-hydroxy acid esters. Solubility was determined by adding various amounts of the drug to a composition including alpha-hydroxy acid esters, mixing, and then visually determining the solubility. The composition selected for the screening was Imwitor^{™} 380, a partially neutralized mixture of citric acid/lactic acid esters of partial glycerides. Imwitor^{™} 380 contains approximately 60% citric acid/lactic acid esters of partial glycerides, less than 32%
1-monoglyceride, and not more than 5% glycerol (Product Information Sheet, 26.13.199e/07.98; Sasol).

Cyclosporine, USP (Abbott), valproic acid (Sigma-Aldrich), fenofibrate (Sigma), and clofibrate (Sigma-Aldrich) were individually added to partially neutralized Imwitor^{™} 380 (Sasol, Witten, Germany) to provide various drug:Imwitor^{™} 380 mixtures. Mixtures were based on weight/weight and ranged from 5% drug/95% Imwitor^{™} 380 to 15% drug/85% Imwitor^{™} 380. The samples were prepared in 12 ml glass vials, containing a stirbar to ensure adequate mixing, and sealed with Teflon lined caps (National Scientific Co.; vial: part no B7800-12; cap: 15-425). Vials were tumbled end-over-end for either 65 or 96 hours using a VanKel^{™} rotating apparatus at 3 rpm and a water bath set at 25°C and then observed visually to assess the solubility of the drug. Table 1 shows the results of the solubility screening assay using partially neutralized Imwitor^{™} 380.

**Table 1**

| **Drug Active/Prodrug** | **Solubility** |
|---|---|
| Cyclosporine | > 150 mg/gm |
| Valproic Acid | > 150 mg/gm |
| Fenofibrate | > 50 mg/gm |
| Clofibrate | > 150 mg/gm |

These solubility results indicate that Imwitor^{™} 380 (a partially neutralized mixture of citric acid/lactic acid esters of partial glycerides) is a suitable solvent for several structurally and therapeutically unrelated drug compounds.

### Example 2

### Solubility Screening

Cyclosporine, USP (Abbott), valproic acid (Sigma-Aldrich), and clofibrate (Sigma-Aldrich) were individually added to unneutralized Imwitor^{™} 380 (Sasol, Witten, Germany) to provide various drug:unneutralized Imwitor^{™} 380 mixtures. Mixtures were based on weight/weight and ranged from 5% drug/95% unneutralized Imwitor^{™} 380 to 10% drug/90% unneutralized Imwitor^{™} 380. The samples were prepared in scintillation vials (Research Products International) containing a stirbar to ensure adequate mixing. Vials were tumbled end-over-end for approximately 24 hours at 7 rpm using a VanKel^{™} rotating apparatus at ambient conditions and then observed visually to assess the solubility of the drug. Table 2 shows the results of the solubility screening assay using unneutralized Imwitor^{™} 380.

**Table 2**

| **Drug Active/Prodrug** | **Solubility** |
|---|---|
| Cyclosporine | > 100 mg/gm |
| Valproic Acid | > 100 mg/gm |
| Clofibrate | > 100 mg/gm |

These solubility results indicate that Imwitor^{™} 380 (an unneutralized mixture of citric acid/lactic acid esters of partial glycerides) is a suitable solvent for several structurally and therapeutically unrelated drug compounds.

### Example 3

### Phase Behavior of an Example Delivery Composition with Changing pH

The phase behavior of Imwitor^{™} 380 unneutralized in combination with either 0.1 *N* HCl (J.T. Baker) or a 50 mM pH 7.4 phosphate buffer was determined. Approximately 3 gram samples were prepared by weight from 0 -100% of each component and vortex mixed. Over a period of several weeks, the samples underwent repeated cycles of equilibration at room temperature and centrifugation (Jouan, Inc. Model GT-244) until the phases were clearly separated with a sharp boundary. Visual observations of the phases were made using crossed polarized plastic sheets to identify birefringent phases, and relative phase volumes were estimated using a ruler to measure the dimensions of the phases. Microscopic observations of select samples were made using a Nikon E600Pol polarizing light microscope.

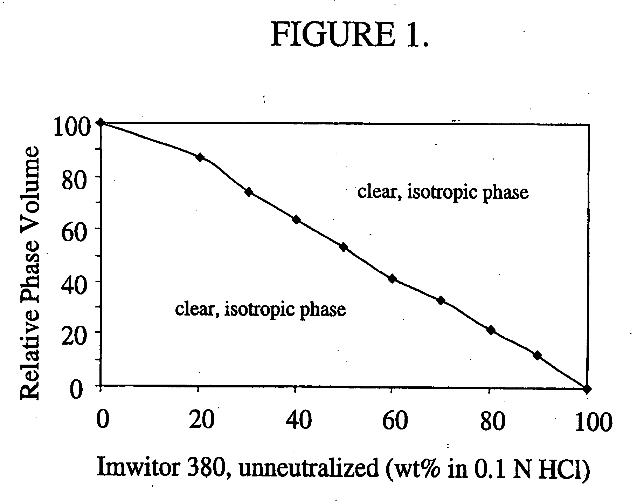

The phase behavior of unneutralized Imwitor^{™} 380 with 0.1 *N* HCl is depicted in Figure 1. As shown in Figure 1, samples prepared with 0.1 *N* HCl consisted of two isotropic phases in equilibrium. The relative volumes of the two phases are essentially the same as the amount of the individual components added. Such data indicates that little miscibility exists between the unneutralized ester and the aqueous phase at pH 1.

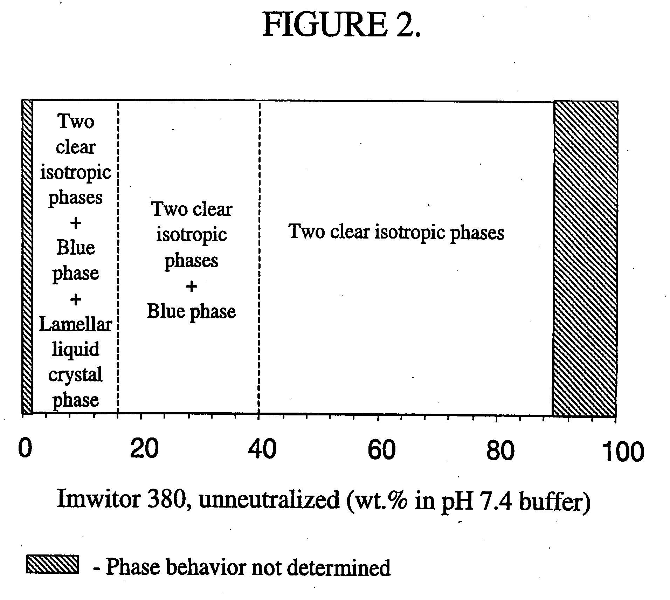

At pH 7.4, two additional phases were identified. A lamellar liquid crystal was identified by its birefringence through crossed polarized plastic sheets and a distinctive pattern observed under the microscope. A second "blue" phase also was identified. The blue phase was stable under repeated centrifugation, however, when viewed under the microscope the blue phase consisted of a dispersion of large droplets, indicating that the phase may not be a single equilibrium phase. Figure 2 shows the type and number of phases observed as a function of the amount (% w/w) unneutralized Imwitor^{™} 380.

As shown by the data, the phase diagrams in Figures 1 and 2 illustrate a significant change in the phase behavior of an example delivery composition as a function of pH.

### Example 4

### Microscopic Analysis of Dispersions of an Example Delivery Composition

Transmission electron microscopy (TEM) was utilized to examine the delivery composition dispersed in various media using a dissolution apparatus. Microscopy samples were removed and stained for TEM.

Samples were prepared by dispersing either 0.5 ml or 5 ml of partially neutralized Imwitor^{™} 380 in 400 ml of distilled water or 0.1 ***N*** HCl using a dissolution USP apparatus 2, paddle system at 37°C, at 150 rpm. TEM samples were removed after 30 minutes of mixing. Grids were prepared by sequentially floating a formvar/carbon filmed grid on a 50 µl drop of the sample for one minute, a drop of stain (2% phosphotungstic acid (pH 7) or 2% aqueous uranyl acetate) for one minute, and two drops of water for 10 seconds each. Excess liquid was blotted off the grid between each step and grids were blotted to dryness after the last step. Each grid was examined with a Philips CM12 TEM operating at 80kV accelerating voltage.

Results from staining with phosphotungstic acid are provided. Observations of the TEM images are summarized in Table 3 below.

**Table 3**

| **Delivery** **Composition** **(volume/400 ml)** | **Dispersion Medium** | **Micrographic Description of Dispersed Delivery Composition** |
|---|---|---|
| Imwitor^{™} 380, partially neutralized; (0.5 ml) | Distilled water | Numerous spherical particles with a few multilamellar structures |
| Imwitor^{™} 380, partially neutralized; (5 ml) | Distilled water | Numerous spherical particles with a few multilamellar structures |
| Imwitor^{™} 380, partially neutralized; (0.5 ml) | 0.1 *N* HCl | Few poorly organized spherical structures |
| Imwitor^{™} 380, partially neutralized; (5 ml) | 0.1 *N* HCl | Numerous poorly organized spherical structures, a few elongated structures, and numerous very small spherical structures |

An example delivery composition dispersed in distilled water formed small particles and lamellar structures, while samples dispersed in acidic media did not form lamellar structures. The TEM data indicates that the example delivery composition forms lamellar vesicles at neutral pH, and does not form lamellar structures at low pH.

### Example 5

### Soft Elastic Capsule Compatibility

Soft elastic capsule airfills (i.e., empty capsules) were filled with an example delivery composition and tested at various time intervals for physical compatibility. Filled capsules were stored for a period of time before performing physical testing.

Soft elastic capsule gelatin shell airfills (L1.25DDXHBHM; R. P. Scherer) were filled with partially neutralized Imwitor^{™} 380 by removing the tip of the capsule and transferring the delivery composition to the interior of the airfill shell via a syringe. The tip was then heat-sealed. The delivery composition-filled soft elastic capsules were placed into sealed vials and stored at 5°C and 40°C. Vials were sampled at various time intervals and physical observations of the capsule shell recorded. Soft elastic capsules filled with Heavy Mineral Oil (USP), stored at the same temperatures and sampled at the same time intervals, were used as controls. Table 4 shows the results of the physical testing of soft elastic capsule airfills filled with partially neutralized Imwitor^{™} 380 at 5°C and Table 5 shows the results of the physical testing at 40°C at various time intervals.

**Table 4**

| **Interval** | **Physical Observations** |
|---|---|
| Initial | SEC intact; pliable; no dimples |
| 2 Weeks | SEC intact; pliable; no dimples |
| 4 Weeks | SEC intact; pliable; no dimples |
| 6 Weeks | SEC intact; pliable; no dimples |
| 8 Weeks | SEC intact; pliable; no dimples |
| 12 Weeks | SEC intact; pliable; no dimples |
| 16 Weeks | SEC intact; pliable; no dimples |
| 20 Weeks | SEC intact; pliable; no dimples; SEC harder than control |
| 25 Weeks | SEC intact; pliable; no dimples; SEC harder than control |

| | |
|---|---|
| SEC = Soft Elastic or Gelatin Capsule | |

**Table 5**

| **Interval** | **Physical Observations** |
|---|---|
| Initial | SEC intact; pliable; no dimples |
| 2 Weeks | SEC intact; pliable; no dimples |
| 4 Weeks | SEC intact; pliable; no dimples |
| 6 Weeks | SEC intact; slightly pliable; no dimples |
| 8 Weeks | SEC intact; slightly pliable; no dimples |
| 12 Weeks | SEC intact; slightly pliable; no dimples |
| 16 Weeks | SEC intact; slightly pliable; no dimples |
| 20 Weeks | SEC intact; slightly pliable; no dimples |
| 25 Weeks | SEC intact; very slightly pliable; no dimples |

| | |
|---|---|
| SEC = Soft Elastic or Gelatin Capsule | |

As shown above, the data indicates that an example delivery composition demonstrates reasonable compatibility with soft elastic capsule gelatin shells.

## Claims

1. A pharmaceutical composition comprising:
a) a carrier composition; and
b) a pharmaceutical drug active,
wherein the carrier composition comprises a delivery composition comprising:
i) an alpha-hydroxy acid ester and
ii) an acylglycerol;
wherein the ester consists essentially of at least one alpha-hydroxy acid portion, at least one glycerol portion, and at least one fatty acid portion; and
wherein the alpha-hydroxy acid ester is found at more than 36% weight/weight (w/w) in the carrier composition.

2. The pharmaceutical composition of claim 1 wherein the carrier composition comprises a delivery composition and a cosolvent.

3. The Pharmaceutical composition of claim 2 wherein the cosolvent is hydrophilic.

4. The pharmaceutical composition of claim 3 wherein the cosolvent is selected from the group consisting of ethanol, benzyl alcohol, propylene glycol, polyethylene glycols, glycerin, water, and N-methyl-2-pyrrolidone, or a mixture thereof.

5. The pharmaceutical composition of claim 2 wherein the cosolvent is lipophilic.

6. The pharmaceutical composition of claim 5 wherein the cosolvent is selected from the group consisting of triacetin, ethyl oleate, long chain alcohols, and isopropyl myristate, or a mixture thereof.

7. The pharmaceutical composition of claim 1 wherein the alpha-hydroxy acid ester is formed by the esterification of monoacylglycerols, diacylglycerols, or a combination thereof, with one or more alpha-hydroxy acids, preferably said alpha-hydroxy acid ester being formed by the esterification of monoacylglycerols with one or more alpha-hydroxy acids, and wherein said monoacylglycerols are obtained from natural plant or animal sources.

8. The pharmaceutical composition of claim 7 wherein said monoacylglycerols are obtained from the group consisting of coconut oil, palm kernel oil, and soybean oil, preferably wherein the alpha-hydroxy acid ester is formed by the esterification of monoacylglycerols with lactic acid, citric acid, or both.

9. The pharmaceutical composition of claim 1 wherein the fatty acid portion of the alpha-hydroxy acid ester comprises unsaturated fatty acid chains, saturated fatty acid chains, or a combination thereof.

10. The pharmaceutical composition of claim 9 wherein the fatty acid portion of the alpha-hydroxy acid ester composes saturated or unsaturated fatty acid chains in the range of C₆-C₂₄ in length, preferably in the range of C₈-C₁₈ in length.

11. The pharmaceutical composition of claim 1 wherein the alpha-hydroxy acid portion of the alpha-hydroxy acid ester is lactic acid, citric acid, or a combination thereof.

12. The pharmaceutical composition of claim 1 wherein the alpha-hydroxy acid ester is unneutralized.

13. The pharmaceutical composition of claim 1 wherein the alpha-hydroxy acid ester is partially neutralized.

14. The pharmaceutical composition of claim 1 wherein the delivery composition is selected from the group consisting of Imwitor^{™} 370, Imwitor^{™} 375, Imwitor^{™} 377, and Imwitor^{™} 380, wherein Imwitor^{™} 380 and 377 are partially neutralized citric acid/lactic acid esters of partial glycerides derived from coconut oil, Imwitor^{™} 375 is a partially neutralized citric acid/lactic acid ester of linoleate and oleate-containing mono- and diglycerides and Imwitor^{™} 370 is a partially neutralized citric acid ester of stearate-containing mono- and diglycerides.

15. The pharmaceutical composition of claim 14 wherein the delivery composition consists essentially of Imwitor^{™} 380.

16. The pharmaceutical composition of claim 15 wherein the Imwitor^{™} 380 is unneutralized.

17. The pharmaceutical composition of claim 16 further comprising a soft elastic capsule shell wherein the carrier composition and the pharmaceutical drug active or prodrug are contained within the capsule shell.

18. The pharmaceutical composition of claim 1 wherein the pharmaceutical drug active is a hydrophobic drug.

19. The pharmaceutical composition of claim 1, wherein the delivery composition imparts lipophilic properties in a medium wherein the pH is less than pH 4, preferably wherein the pH is pH 1-3.

20. The pharmaceutical composition of claim 1, wherein the delivery composition imparts surface active properties in a medium wherein the pH is pH 6-8.

21. A pharmaceutical composition according to Claim 1 wherein the alpha-hydroxy acid ester is found at 60% w/w or more in the carrier composition.

22. A pharmaceutical composition according to Claim 1 wherein the carrier composition consists entirely of the delivery composition.

23. A pharmaceutical composition according to claim 1 comprising:
a) a delivery composition comprising:
i) an alpha-hydroxy acid ester and
ii) an acylglycerol;
wherein the ester consists essentially of at least one alpha-hydroxy acid portion, at least one glycerol portion, and at least one fatty acid portion; and wherein the alpha-hydroxy acid ester is found at 60% w/w or more in the delivery composition; and
b) a pharmaceutical drug active.

24. A pharmaceutical composition according to claim 1 comprising:
a) a carrier composition consisting essentially of
1) a delivery composition comprising:
i) an alpha-hydroxy acid ester, wherein the ester consists essentially of at least one alpha hydroxy acid portion, at least one glycerol portion, and at least one fatty acid portion; and
ii) an acylglycerol; and
2) a cosolvent; and
b) a pharmaceutical drug active.

25. A pharmaceutical capsule comprising:
1) a capsule shell and
2) a fill composition comprising a composition according to any one of claims 1-24.

26. A method for enhancing absorbability of a pharmaceutical drug comprising the step of:
preparing a pharmaceutical composition according to any one of claims 1-24 for oral administration.

27. A composition according to any one of claims 1-25 for use in therapy by oral administration.

## Patentansprüche

1. Eine pharmazeutische Zusammensetzung, die folgendes umfaßt:
a) eine Trägerzusammensetzung und
b) einen pharmazeutisch wirksamen Arzneistoff,
worin die Trägerzusammensetzung eine Zuführungszusammensetzung umfaßt, die folgendes umfaßt:
i) einen alpha-Hydroxycarbonsäureester und
ii) ein Acylglycerol;
worin der Ester im wesentlichen aus mindestens einem alpha-Hydroxycarbonsäureanteil, mindestens einem Glycerolanteil und mindestens einem Fettsäureanteil besteht und worin der alpha-Hydroxycarbonsäureester mit mehr als 36% Masse/Masse (m/m) in der Trägerzusammensetzung zu finden ist.

2. Die pharmazeutische Zusammensetzung gemäß Anspruch 1, worin die Trägerzusammensetzung eine Zuführungszusammensetzung und ein Cosolvens umfaßt.

3. Die pharmazeutische Zusammensetzung gemäß Anspruch 2, worin das Cosolvens hydrophil ist.

4. Die pharmazeutische Zusammensetzung gemäß Anspruch 3, worin das Cosolvens gewählt ist aus der Gruppe bestehend aus Ethanol, Benzylalkohol, Propylenglykol, Polyethylenglykolen, Glycerin, Wasser und N-Methyl-2-pyrrolidon, oder eine Mischung davon.

5. Die pharmazeutische Zusammensetzung gemäß Anspruch 2, worin das Cosolvens lipophil ist.

6. Die pharmazeutische Zusammensetzung gemäß Anspruch 5, worin das Cosolvens gewählt ist aus der Gruppe bestehend aus Triacetin, Ethyloleat, langkettigen Alkoholen und Isopropylmyristat, oder eine Mischung davon.

7. Die pharmazeutische Zusammensetzung gemäß Anspruch 1, worin der alpha-Hydroxycarbonsäureester durch die Veresterung von Monoacylglycerolen, Diacylglycerolen oder einer Kombination davon mit einer oder mehreren alpha-Hydroxycarbonsäuren gebildet wird, wobei der alpha-Hydroxycarbonsäureester vorzugsweise durch die Veresterung von Monoacylglycerolen mit einer oder mehreren alpha-Hydroxycarbonsäuren gebildet wird und worin die Monoacylglycerole aus natürlichen pflanzlichen oder tierischen Quellen gewonnen werden.

8. Die pharmazeutische Zusammensetzung gemäß Anspruch 7, worin die Monoacylglycerole aus der Gruppe bestehend aus Kokosöl, Palmkernöl und Sojabohnenöl gewonnen werden, worin der alpha-Hydroxycarbonsäureester vorzugsweise durch die Veresterung von Monoacylglycerolen mit Milchsäure, Zitronensäure oder beidem gebildet wird.

9. Die pharmazeutische Zusammensetzung gemäß Anspruch 1, worin der Fettsäureanteil des alpha-Hydroxycarbonsäureesters ungesättigte Fettsäureketten, gesättigte Fettsäureketten oder eine Kombination davon umfaßt.

10. Die pharmazeutische Zusammensetzung gemäß Anspruch 9, worin der Fettsäureanteil des alpha-Hydroxycarbonsäureesters gesättigte oder ungesättigte Fettsäureketten in dem Längen-Bereich von C₆-C₂₄, vorzugsweise in dem Längen-Bereich von C₈-C₁₈, umfaßt.

11. Die pharmazeutische Zusammensetzung gemäß Anspruch 1, worin der alpha-Hydroxycarbonsäureanteil des alpha-Hydroxycarbonsäureesters Milchsäure, Zitronensäure oder eine Kombination davon ist.

12. Die pharmazeutische Zusammensetzung gemäß Anspruch 1, worin der alpha-Hydroxycarbonsäureester nicht neutralisiert ist.

13. Die pharmazeutische Zusammensetzung gemäß Anspruch 1, worin der alpha-Hydroxycarbonsäureester teilweise neutralisiert ist.

14. Die pharmazeutische Zusammensetzung gemäß Anspruch 1, worin die Zuführungszusammensetzung gewählt ist aus der Gruppe bestehend aus Imwitor^{™} 370, Imwitor^{™} 375, Imwitor^{™} 377 und Imwitor^{™} 380, worin Imwitor^{™} 380 und 377 teilweise neutralisierte Zitronensäure-/Milchsäureester von unvollständig veresterten Glyceriden, abgeleitet von Kokosöl, sind, Imwitor^{™} 375 ein teilweise neutralisierter Zitronensäure-/Milchsäureester von Linoleat- und Oleat-enthaltenden Mono- und Diglyceriden ist und Imwitor^{™} 370 ein teilweise neutralisierter Zitronensäureester von Stearat-enthaltenden Mono- und Diglyceriden ist.

15. Die pharmazeutische Zusammensetzung gemäß Anspruch 14, worin die Zuführungszusammensetzung im wesentlichen aus Imwitor^{™} 380 besteht.

16. Die pharmazeutische Zusammensetzung gemäß Anspruch 15, worin das Imwitor^{™} 380 nicht neutralisiert ist.

17. Die pharmazeutische Zusammensetzung gemäß Anspruch 16, die weiter eine weiche elastische Kapselhülle umfaßt, worin die Trägerzusammensetzung und der pharmazeutisch wirksame Arzneistoff oder das Prodrug innerhalb der Kapselhülle enthalten sind.

18. Die pharmazeutische Zusammensetzung gemäß Anspruch 1, worin der pharmazeutisch wirksame Arzneistoff ein hydrophober Arzneistoff ist.

19. Die pharmazeutische Zusammensetzung gemäß Anspruch 1, worin die Zuführungszusammensetzung lipophile Eigenschaften in einem Medium verleiht, worin der pH niedriger als pH 4 ist, vorzugsweise pH 1-3.

20. Die pharmazeutische Zusammensetzung von Anspruch 1, worin die Zuführungszusammensetzung oberflächenaktive Eigenschaften verleiht in einem Medium, worin der pH pH 6-8 ist.

21. Eine pharmazeutische Zusammensetzung gemäß Anspruch 1, worin der alpha-Hydroxysäureester bei 60% m/m oder mehr in der Trägerzusammensetzung gefunden wird.

22. Eine pharmazeutische Zusammensetzung gemäß Anspruch 1, worin die Trägerzusammensetzung vollständig aus der Zuführungszusammensetzung besteht.

23. Eine pharmazeutische Zusammensetzung gemäß Anspruch 1, die folgendes umfaßt:
a) eine Zuführungszusammensetzung, die folgendes umfaßt:
i) einen alpha-Hydroxysäureester und
ii) ein Acylglycerol;
worin der Ester im wesentlichen aus mindestens einem alpha-Hydroxysäureanteil, mindestens einem Glycerolanteil und mindestens einem Fettsäureanteil besteht; und worin der alpha-Hydroxysäureester bei 60% m/m oder mehr in der Zuführungszusammensetzung gefunden wird; und
b) einen pharmazeutisch aktiven Arzneistoff.

24. Eine pharmazeutische Zusammensetzung gemäß Anspruch 1, die folgendes umfaßt:
a) eine Trägerzusammensetzung die im wesentlichen aus folgendem besteht:
1) einer Zuführungszusammensetzung, die folgendes umfaßt:
i) einen alpha-Hydroxysäureester, worin der Ester im wesentlichen aus mindestens einem alpha-Hydroxysäureanteil, mindestens einem Glycerolanteil und mindestens einem Fettsäureanteil besteht; und
ii) einem Acylglycerol; und
2) ein Cosolvens; und
b) einen pharmazeutisch aktiven Arzneistoff.

25. Eine pharmazeutische Kapsel, die folgendes umfaßt:
1) eine Kapselhülle und
2) eine Füllzusammensetzung umfassend eine Zusammensetzung gemäß irgendeinem der Ansprüche 1-24.

26. Ein Verfahren zur Verbesserung der Absorbierbarkeit eines pharmazeutischen Arzneistoffs, umfassend den Schritt:
Herstellen einer pharmazeutischen Zusammensetzung gemäß irgendeinem der Ansprüche 1-24 für die orale Verabreichung.

27. Eine Zusammensetzung gemäß irgendeinem der Ansprüche 1-25 für die Verwendung in der Therapie durch orale Verabreichung.

## Revendications

1. Composition pharmaceutique comprenant :
a) une composition de support ; et
b) un principe actif de médicament pharmaceutique,
dans lequel la composition de support comprend une composition d'administration comprenant :
i) un ester d'alpha-hydroxy acide et
ii) un acide glycérol ;
dans laquelle l'ester est essentiellement constitué d'au moins une portion acide alpha-hydroxy, d'au moins une portion glycérol et d'au moins une portion acide gras ; et
dans laquelle l'ester d'alpha-hydroxy acide se trouve à plus de 36 % poids/poids (p/p) dans la composition de support.

2. Composition pharmaceutique selon la revendication 1, dans laquelle la composition de support comprend une composition d'administration et un co-solvant.

3. Composition pharmaceutique selon la revendication 2, dans laquelle le co-solvant est hydrophile.

4. Composition pharmaceutique selon la revendication 3, dans laquelle le co-solvant est choisi dans le groupe consistant en l'éthanol, l'alcool benzylique, le propylène glycol, les poly(éthylène glycols), la glycérine, l'eau et la N-méthyl-2-pyrrolidone ou un mélange de ceux-ci.

5. Composition pharmaceutique selon la revendication 2, dans laquelle le co-solvant est lipophile.

6. Composition pharmaceutique selon la revendication 5, dans laquelle le co-solvant est choisi dans le groupe consistant en la triacétine, l'oléate d'éthyle, les alcools à longue chaîne et le myristate d'isopropyle, ou un mélange de ceux-ci.

7. Composition pharmaceutique selon la revendication 1, dans laquelle l'ester d'alpha-hydroxy acide est formé par l'estérification de monoacylglycérols, diacylglycérols, ou une combinaison de ceux-ci, avec un ou plusieurs alpha-hydroxy acides, de préférence ledit ester d'alpha-hydroxy acide est formé par l'estérification de monoacylglycérols avec un ou plusieurs alpha-hydroxy acides, et dans laquelle lesdits monoacylglycérols sont obtenus à partir de sources végétales ou animales naturelles.

8. Composition pharmaceutique selon la revendication 7, dans laquelle lesdits monoacylglycérols sont obtenus dans le groupe consistant en l'huile de noix de coco, l'huile de palmiste, l'huile de soja, de préférence dans laquelle l'ester d'alpha-hydroxy acide est formé par l'estérification de monoacylglycérols avec l'acide lactique, l'acide citrique ou les deux.

9. Composition pharmaceutique selon la revendication 1, dans laquelle la portion acide gras de l'ester d'alpha-hydroxy acide comprend des chaînes d'acides gras insaturés, des chaînes d'acides gras saturés ou une combinaison de celles-ci.

10. Composition pharmaceutique selon la revendication 9, dans laquelle la portion acide gras de l'ester d'alpha-hydroxy acide comprend des chaînes d'acides gras saturés ou insaturés d'une longueur dans la gamme de C₆ à C₂₄, de préférence d'une longueur dans la gamme de C₈ à C₁₈.

11. Composition pharmaceutique selon la revendication 1, dans laquelle la portion alpha-hydroxy acide de l'ester d'alpha-hydroxy acide est l'acide lactique, l'acide citrique ou une combinaison de ceux-ci.

12. Composition pharmaceutique selon la revendication 1, dans laquelle l'ester d'alpha-hydroxy acide est non neutralisé.

13. Composition pharmaceutique selon la revendication 1, dans laquelle l'ester d'alpha-hydroxy acide est partiellement neutralisé.

14. Composition pharmaceutique selon la revendication 1, dans laquelle la composition d'administration est choisie dans le groupe consistant en Imwitor^{™} 370, Imwitor^{™} 375, Imwitor^{™} 377 et Imwitor^{™} 380, où Imwitor^{™} 380 et 377 sont des esters d'acide citrique/acide lactique partiellement neutralisés de glycérides partiels dérivés d'huile de noix de coco, Imwitor^{™} 375 est un ester d'acide citrique/acide lactique partiellement neutralisé de linoléate et de mono- et di-glycérides contenant de l'oléate et Imwitor^{™} 370 est un ester d'acide citrique partiellement neutralisé de mono- et di-glycérides contenant du stéarate.

15. Composition pharmaceutique selon la revendication 14, dans laquelle la composition d'administration est essentiellement constituée d'Imwitor^{™} 380.

16. Composition pharmaceutique selon la revendication 15, dans laquelle l'Imwitor^{™} 380 est non neutralisé.

17. Composition pharmaceutique selon la revendication 16, comprenant en outre une carapace de capsule élastique molle, dans laquelle la composition de support et le principe actif de médicament pharmaceutique ou promédicament sont contenus à l'intérieur de la carapace de capsule.

18. Composition pharmaceutique selon la revendication 1, dans laquelle le principe actif de médicament pharmaceutique est un médicament hydrophobe.

19. Composition pharmaceutique selon la revendication 1, dans laquelle la composition d'administration communique des propriétés lipophiles dans un milieu dans lequel le pH est inférieur à 4, de préférence dans lequel le pH est de 1 à 3.

20. Composition pharmaceutique selon la revendication 1, dans laquelle la composition d'administration communique des propriétés tensioactives dans un milieu dans lequel le pH est de 6 à 8.

21. Composition pharmaceutique selon la revendication 1, dans laquelle l'ester d'alpha-hydroxy acide se trouve à hauteur de 60 % p/p ou plus dans la composition de support.

22. Composition pharmaceutique selon la revendication 1, dans laquelle la composition de support est entièrement constituée de la composition d'administration.

23. Composition pharmaceutique selon la revendication 1, comprenant :
a) une composition d'administration comprenant :
i) un ester d'alpha-hydroxy acide et
ii) un acide glycérol ;
dans laquelle l'ester est essentiellement constitué d'au moins une portion alpha-hydroxy acide, au moins une portion glycérol et au moins une portion acide gras ; et
dans laquelle l'ester d' alpha-hydroxy acide se trouve à hauteur de 60 % p/p ou plus dans la composition d'administration ; et
b) un principe actif de médicament pharmaceutique.

24. Composition pharmaceutique selon la revendication 1, comprenant :
a) une composition de support essentiellement constituée
1) d'une composition d'administration comprenant :
i) d'un ester d'alpha-hydroxy acide, dans laquelle l'ester est essentiellement constitué d'au moins une portion alpha-hydroxy acide, au moins une portion glycérol et au moins une portion acide gras ; et
ii) d'un acide glycérol ; et
2) d'un co-solvant ; et
b) d'un principe actif de médicament pharmaceutique.

25. Capsule pharmaceutique comprenant :
1) une carapace de capsule et
2) une composition de remplissage comprenant une composition selon l'une quelconque des revendications 1 à 24.

26. Procédé pour accentuer l'absorbabilité d'un médicament pharmaceutique comprenant l'étape consistant à :
préparer une composition pharmaceutique selon l'une quelconque des revendications 1 à 24 pour administration orale.

27. Composition selon l'une quelconque des revendications 1 à 25, à utiliser en thérapie par administration orale.
